# EUROPÄISCHE PATENTSCHRIFT

(11) **EP 2 303 465 B1**
(45) Veröffentlichungstag und Bekanntmachung des Hinweises auf die Patenterteilung: **16.12.2015**
(21) Anmeldenummer: 09765620.1
(22) Anmeldetag: 18.06.2009
(51) Int. Cl.: B05B 7/04, B05B 13/06

(54) **BESCHICHTUNGSVORRICHTUNG**
COATING APPARATUS
DISPOSITIF DE REVÊTEMENT

(30) Priorität: 19.06.2008 DE 102008030272
(43) Veröffentlichungstag der Anmeldung: 06.04.2011
(73) Patentinhaber: Arzneimittel GmbH Apotheker Vetter & Co. Ravensburg, 88212 Ravensburg (DE)
(72) Erfinder: BÖTTGER, Frank, 88214 Ravensburg (DE); SPÄTH, Günther, 88069 Tettnang (DE)
(74) Vertreter: Gleiss, Alf-Olav
(86) Internationale Anmeldenummer: PCT/EP2009/004386
(87) Internationale Veröffentlichungsnummer: WO 2009/153040

(56) Entgegenhaltungen:
- WO-A-2007/100838
- DE-A1- 10 126 100
- DE-C1- 10 036 832
- DE-U1- 29 714 564
- US-A- 4 164 325

## Beschreibung

Die Erfindung betrifft eine Beschichtungsvorrichtung gemäß Oberbegriff des Anspruchs 1. Siehe zum Beispiel DE 100 36832.

Derartige Beschichtungsvorrichtungen sind bekannt. Häufig werden Hohlkörper auf ihrer Innenseite mit einem zerstäubten Fluid beschichtet, indem der Hohlkörper mit einer Öffnung über eine Zerstäubungsdüse gehalten wird. Die Zerstäubungsdüse zerstäubt das Fluid und erzeugt so ein Aerosol, also ein Gemisch aus Treibgas, beispielsweise Luft, und aus aus dem Fluid gebildeten Schwebeteilchen beziehungsweise Fluidtröpfchen. Dieses Aerosol wird durch die Öffnung in den Hohlkörper eingeblasen, woraufhin sich die darin enthaltenen Fluidtröpfchen auf der Innenseite des Hohlkörpers absetzen können. Dabei ist die Zerstäubung des Fluids durch die Düse von entscheidender Bedeutung für eine gleichmäßige Verteilung der Fluidtröpfchen auf der Innenseite des Hohlkörpers, was oft nicht gelingt.

Es ist daher Aufgabe der Erfindung, eine Beschichtungsvorrichtung mit den in Oberbegriff des Anspruchs 1 genannten Merkmalen zu schaffen, welche eine gleichmäßige Verteilung des zerstäubten Fluids auf der Innenseite des Hohlkörpers, insbesondere durch eine verbesserte Zerstäubung des Fluids, ermöglicht.

Zur Lösung dieser Aufgabe wird eine Beschichtungsvorrichtung vorgeschlagen, welche die in Anspruch 1 genannten Merkmale aufweist. Sie zeichnet sich durch mindestens ein einen Zerstäubungskanal einschließendes Zerstäubungsrohr aus, in das unter einem Überdruck stehendes Treibgas zum Zerstäuben eines unzerstäubten Fluids einleitbar ist und das mindestens eine Austrittsöffnung aufweist. Sie umfasst mindestens eine eine Ausbringöffnung aufweisende Hohlnadel für das unzerstäubte Fluid, die mit dem Zerstäubungskanal zusammenwirkt und im Wesentlich koaxial zu diesem angeordnet ist, wobei das Zerstäubungsrohr und die Hohlnadel eine Venturi-Anordnung bilden. Mit einem Druck beaufschlagtes Treibgas wird also in das Zerstäubungsrohr beziehungsweise den darin angeordneten Zerstäubungskanal eingeleitet. Das unzerstäubte Fluid wird über eine Hohlnadel eingebracht, die mit dem Zerstäubungskanal zusammenwirkt. Das bedeutet, dass das unzerstäubte Fluid durch ein Zusammenwirken von Zerstäubungskanal und Hohlnadel zerstäubt wird. Dies wird im Wesentlichen durch das durch das Zerstäubungsrohr beziehungsweise den Zerstäubungskanal strömende Treibgas erreicht. Dabei kann ein beliebiges Mengenverhältnis von unzerstäubtem Fluid zu Treibgas vorgesehen sein, vorzugsweise wird jedoch mehr Treibgas als Fluid in die Beschichtungsvorrichtung eingebracht. Das unzerstäubte Fluid tritt durch die Ausbringöffnung der Hohlnadel aus. Die Ausbringöffnung ist so angeordnet, dass durch das Zusammenwirken von Hohlnadel und Zerstäubungskanal das Fluid zerstäubt werden kann. Die Hohlnadel ist im Wesentlichen koaxial zu dem Zerstäubungskanal angeordnet. Daher kann die Beschichtungsvorrichtung äußerst schlank gebaut sein und insbesondere einen schmalen Querschnitt aufweisen. Durch das Zerstäuben des Fluids liegt ein Aerosol, also ein Gemisch aus dem Treibgas und dem zerstäubten Fluid beziehungsweise den Fluidtröpfchen, vor. Das Aerosol strömt durch das Zerstäubungsrohr, bis es die Austrittsöffnung erreicht, und aus dem Zerstäubungsrohr austreten kann. Die Austrittsöffnung des Zerstäubungsrohrs ist so angeordnet, dass eine sehr gleichmäßige Verteilung des zerstäubten Fluids auf der Innenseite des Hohlkörpers gewährleistet ist.

Die Beschichtungsanordnung zeichnet sich dadurch aus, dass das Zerstäubungsrohr und die Hohlnadel eine Venturi-Anordnung bilden. Das bedeutet, dass eine Anordnung gemäß einer Venturi-Düse beziehungsweise eines Venturi-Rohrs vorliegt. Die Ausbringöffnung der Hohlnadel ist also in einem Bereich mit einem engsten Querschnitt des Zerstäubungsrohrs angeordnet, oder die Hohlnadel bildet diesen engsten Querschnitt gemeinsam mit dem Zerstäubungsrohr. Dabei kann der genannte Querschnitt auch nur einen Bereich des engsten Querschnitts bilden oder ein lokal engster Querschnitt beziehungsweise ein Bereich davon sein. Das bedeutet nicht, dass der Bereich der Venturi-Anordnung insgesamt den engsten Querschnitt, insbesondere des Zerstäubungsrohrs, bilden muss.

In dem Bereich der Venturi-Anordnung liegt eine hohe Strömungsgeschwindigkeit des Treibgases vor. Der Treibgasstrom reißt das unzerstäubte Fluid durch die Ausbringöffnung aus der Hohlnadel heraus, es liegt also ein Impulsaustausch zwischen Treibgas und unzerstäubtem Fluid vor. Dadurch wird das unzerstäubte Fluid beschleunigt. Durch die Beschleunigung und damit höhere Geschwindigkeit entsteht ein Unterdruck, der eine Sogwirkung auf das unzerstäubte Fluid ausübt. Daher kann ein Förderdruck, der zum Fördern des unzerstäubten Fluids durch die Hohlnadel notwendig ist, geringer sein, als der im Bereich der Venturi-Anordnung vorliegende Druck. Anschließend an die Venturi-Anordnung, also in Strömungsrichtung des Aerosols stromabwärts, kann das Fluid beispielsweise durch eine Querschnittserweiterung wieder verzögert werden. Es ist vorgesehen, dass in dem Zerstäubungskanal beziehungsweise dem Zerstäubungsrohr ein kontinuierlicher Strom des Treibgases vorliegt und durch die Hohlnadel ein kontinuierlicher Strom des unzerstäubten Fluids eingebracht wird. Tritt das mit dem Treibgas vermengte zerstäubte Fluid, also das Aerosol, stromabseitig der Venturi-Anordnung durch die Austrittsöffnung des Zerstäubungsrohrs aus, so wird das Aerosol auf Umgebungsdruck entspannt. Während dem dabei ablaufenden Entspannungsvorgang kann im Bereich der Austrittsöffnung eine sehr hohe Strömungsgeschwindigkeit des Aerosols erreicht werden. Insbesondere können die Fluidteilchen des zerstäubten Fluids bis in den Überschallbereich beschleunigt werden. Mit der erfindungsgemäßen Beschichtungsvorrichtung kann eine sehr gute Vermischung von Treibgas und zerstäubtem Fluid, insbesondere sehr kleine Fluidtröpfchen, erzielt werden. In Verbindung mit der sehr hohen Austrittsgeschwindigkeit des Aerosols bei einem Austreten aus der Austrittsöffnung kann eine deutlich verbesserte Homogenität der Verteilung der Fluidtröpfchen auf der Innenseite des Hohlkörpers erreicht werden.

Eine Weiterbildung der Erfindung sieht vor, dass die Hohlnadel zumindest bereichsweise im Zerstäubungskanal angeordnet ist. Das heißt, dass die Hohlnadel teilweise in dem Zerstäubungskanal beziehungsweise durch das Zerstäubungsrohr verläuft. Die Ausbringöffnung der Hohlnadel kann ebenso in dem Zerstäubungskanal vorgesehen sein. In diesem Fall ist die Venturi-Anordnung in dem Zerstäubungskanal realisiert. Es kann auch vorgesehen sein, dass die Hohlnadel zwar innerhalb des Zerstäubungskanals verläuft, die Venturi-Anordnung aber nicht in dem Zerstäubungskanal vorgesehen ist. Vorzugsweise verläuft die Hohlnadel zentrisch in dem Zerstäubungskanal.

Eine Weiterbildung der Erfindung sieht vor, dass eine Verjüngung des Zerstäubungskanals vorgesehen ist. Der Zerstäubungskanal weist also eine Verringerung seines inneren Querschnitts auf. Die Verjüngung kann beispielsweise dazu vorgesehen sein, das Treibgas im Bereich der Venturi-Anordnung beziehungsweise stromaufwärts von dieser zu beschleunigen und damit den statischen Druck zu senken. In diesem Fall bildet die Verjüngung einen Teil der Venturi-Anordnung, indem sie zumindest bereichsweise den engsten Querschnitt bildet. Es können auch mehrere Verjüngungen vorgesehen sein, insbesondere kann eine Gesamtverjüngung des Zerstäubungskanals über mehrere Abschnitte erfolgen.

Eine Weiterbildung der Erfindung sieht vor, dass die Ausbringöffnung der Hohlnadel in einem Bereich der Verjüngung angeordnet ist. Dabei kann die Ausbringöffnung in dem Bereich sowohl stromaufals auch stromabwärts der Verjüngung angeordnet sein. Vorzugsweise bilden das Zerstäubungsrohr und die in dem Bereich der Verjüngung angeordnete Ausbringöffnung der Hohlnadel die Venturi-Anordnung aus.

Eine Weiterbildung der Erfindung sieht vor, dass die Ausbringöffnung in einem Bereich der Austrittsöffnung vorgesehen ist. Die Ausbringöffnung ist also in dem Bereich angeordnet, der - bezogen auf eine Gesamtlänge des Zerstäubungsrohrs beziehungsweise des Zerstäubungskanals - nahe der Austrittsöffnung liegt. Auf diese Weise kann die hohe Strömungsgeschwindigkeit des Aerosols im Bereich der Venturi-Anordnung genutzt werden, um die Beschichtung der Innenseite des Hohlkörpers mit dem zerstäubten Fluid vorzunehmen. Insbesondere sind also keine weiteren konstruktiven Maßnahmen zu ergreifen, um die Strömungsgeschwindigkeit des Aerosols weiter zu erhöhen. Vielmehr kann es vorgesehen sein, stromabwärts der Venturi-Anordnungen einen Diffusor vorzusehen, der für eine Verzögerung der Strömungsgeschwindigkeit, jedoch auch für eine noch wirkungsvollere Zerstäubung des Fluids sorgt.

Eine Weiterbildung der Erfindung sieht vor, dass die mindestens eine Austrittsöffnung frontseitig an dem Zerstäubungsrohr vorgesehen ist. Das bedeutet, dass die Austrittsöffnung in dem am weitesten stromabwärts gelegenen Bereich der Beschichtungsvorrichtung ausgebildet ist. Die Austrittsöffnung verbindet den Zerstäubungskanal fluidtechnisch mit der Umgebung. Durch das Zerstäubungsrohr beziehungsweise den Zerstäubungskanal strömendes Treibgas und/oder Aerosol kann somit durch die Austrittsöffnung austreten. Vorzugsweise tritt das Aerosol beziehungsweise das zerstäubte Fluid durch die Austrittsöffnung im Wesentlichen in axialer Richtung des Zerstäubungsrohrs beziehungsweise in einem axial zu diesem angeordneten Austrittskegel aus.

Eine Weiterbildung der Erfindung sieht vor, dass die mindestens eine Austrittsöffnung in einer Außenwandung des Zerstäubungsrohrs vorgesehen ist. Dabei kann ein Austritt des Aerosols in radialer Richtung des Zerstäubungsrohrs erfolgen. Das Aerosol kann jedoch auch unter einem Winkel zu der radialen Richtung austreten.

Eine Weiterbildung der Erfindung sieht vor, dass mindestens zwei Austrittsöffnungen vorgesehen sind, die in der Außenwandung des Zerstäubungsrohrs vorgesehen sind und die über einen Umfang des Zerstäubungsrohrs verteilt, insbesondere einander diametral gegenüberliegend, angeordnet sind. Durch eine Verteilung der Austrittsöffnungen über den Umfang des Zerstäubungsrohrs kann die Verteilung des durch die Austrittsöffnungen austretenden Aerosols auf der Innenseite des Hohlkörpers beeinflusst werden. Beispielsweise können so in Bereichen der Innenseite größere oder kleinere Mengen des Aerosols für die Beschichtung vorgesehen sein. Zu diesem Zweck können die Austrittsöffnungen unregelmäßig über den Umfang des Zerstäubungsrohrs verteilt sein. Auch eine gleichmäßige, insbesondere diametral gegenüberliegende, Anordnung der Austrittsöffnungen ist möglich. Beispielsweise kann vorgesehen sein, über eine ausreichend hohe Anzahl an Austrittsöffnungen die Gleichmäßigkeit der Beschichtung der Innenseite des Hohlkörpers zu verbessern.

Eine Weiterbildung der Erfindung sieht vor, dass die mindestens eine Austrittsöffnung über zumindest einen Teil einer axialen Erstreckung der Innenseite des Hohlkörpers mittels einer Verlagerungsvorrichtung verlagerbar ist. Die Austrittsöffnung ist also an verschiedenen Positionen innerhalb des Hohlkörpers anordenbar. Das bedeutet insbesondere, dass die Beschichtungsvorrichtung beziehungsweise das Zerstäubungsrohr zumindest bereichsweise in den Hohlkörper eingebracht wird. Vorzugsweise ist vorgesehen, dass die Austrittsöffnung innerhalb des Hohlkörpers während des Beschichtungsvorgangs verlagert wird. Dabei ist eine Verlagerung entweder in den Hohlkörper hinein oder aus ihm heraus möglich. In letzterem Fall wird die Austrittsöffnung zunächst in eine Axialposition innerhalb des Hohlkörpers gebracht, die der Öffnung des Hohlkörpers, durch die ein Einbringen zumindest eines Teils des Zerstäubungsrohrs in den Hohlkörper erfolgt, abgewandt ist. Anschließend wird mit der Beschichtung der Innenseite begonnen, und während des Beschichtungsvorgangs die Austrittsöffnung auf die Öffnung des Hohlkörpers zu verlagert. Vorzugsweise erfolgt eine Verlagerung der Austrittsöffnung während des Beschichtungsvorgangs mit einer gleichmäßigen Geschwindigkeit.

Bei der Verlagerung weist die Austrittsöffnung des Zerstäubungsrohrs während des Beschichtungsvorgangs in einem großen Bereich einen gleichen Abstand zu der Innenseite des Hohlkörpers auf. Somit kann eine besonders gleichmäßige Beschichtung der Innenseite des Hohlkörpers erreicht werden. Die Verlagerung der Austrittsöffnung kann beispielsweise durch eine Verlagerung des Zerstäubungsrohrs gegenüber dem Hohlkörper oder durch eine Verlagerung des Hohlkörpers gegenüber dem Zerstäubungsrohr erreicht werden. Um die Austrittsöffnung auch in schlanke Hohlkörper einbringen zu können, ist es erforderlich, dass die Beschichtungsvorrichtung beziehungsweise der Teil der Beschichtungsvorrichtung, die in den Hohlkörper eingebracht wird, beispielsweise das Zerstäubungsrohr, einen kleinen Querschnitt aufweist. Dies wird, wie oben beschrieben, durch Verwendung der Venturi-Anordnung erreicht, da diese die zur Beschichtung der Innenseite des Hohlkörpers erforderliche Funktion auf sehr kleinem Raum realisiert. Durch die mittels der Venturi-Anordnung erreichte hohe Austrittsgeschwindigkeit des Aerosols aus der Austrittsöffnung ist eine Verlagerung der Austrittsöffnung nicht über einen gesamten Bereich der zu beschichteten Innenseite des Hohlkörpers notwendig. Vorzugsweise wird der Bereich, in welchem eine Verlagerung der Austrittsöffnung erfolgt, auf den zu beschichteten Bereich der Innenseite des Hohlkörpers und eine Zerstäubungscharakteristik der Beschichtungsvorrichtung abgestimmt. Unter Zerstäubungscharakteristik kann beispielsweise die Austrittsgeschwindigkeit und/oder eine charakteristische Größe der Fluidtröpfchen des zerstäubten Fluids verstanden werden. Auch Eigenschaften des zur Beschichtung verwendeten Fluids sind in diese Betrachtung einzubeziehen, beispielsweise dessen Viskosität.

Eine Weiterbildung der Erfindung sieht vor, dass der Hohlkörper während der Beschichtung von einer Halteeinrichtung gehalten ist. Der Hohlkörper wird beispielsweise vor der Beschichtung von der Halteeinrichtung erfasst oder in die Halteeinrichtung eingebracht. Auf diese Weise ist eine stabile, insbesondere schwingungsfreie, Halterung des Hohlkörpers während der Beschichtung gewährleistet. Der Hohlkörper wird während der Beschichtung von der Halteeinrichtung gehalten und nach dem Beschichtungsvorgang aus ihr entnommen beziehungsweise von der Halteeinrichtung losgelassen. Vorzugsweise kann die Halteeinrichtung klammerförmig ausgebildet sein. Das bedeutet, dass der Hohlkörper zumindest bereichsweise von der Halteeinrichtung umfasst ist, während er von ihr gehalten ist.

Eine Weiterbildung der Erfindung sieht vor, dass der Hohlkörper in die Halteeinrichtung einclipsbar beziehungsweise durch Clipswirkung darin gehalten ist. Vorzugsweise ist vorgesehen, dass der Hohlkörper in einem elastischen Bereich der Halteeinrichtung in diese einclipsbar ist. Das bedeutet, dass der Hohlkörper in die Halteeinrichtung eingedrückt wird, wobei ein elastisch ausgebildeter Bereich der Halteeinrichtung zurückweicht. Ist der Hohlkörper in der Halteeinrichtung positioniert, bewegt sich der verformte Bereich durch seine elastische Ausbildung zumindest im Wesentlichen wieder in seine ursprüngliche Position zurück, womit der Hohlkörper in die Halteeinrichtung eingeclipst ist. Durch die beschriebene Clipswirkung ist der Hohlkörper in der Halteeinrichtung fest, insbesondere nicht axial und/oder radial verschiebbar, gehalten (in Bezug auf die Halteeinrichtung). Das Halten des Hohlkörpers in der Halteeinrichtung durch Clipswirkung erlaubt es, die Halteeinrichtung ohne mechanisch bewegte Teile und damit äußerst preisgünstig auszugestalten. Alternativ kann die Clipswirkung mittels Federkraft auf einen beweglich, insbesondere schwenkbeweglich, gelagerten Bereich der Halteeinrichtung erzielt werden.

Eine Weiterbildung der Erfindung sieht vor, dass der Hohlkörper mittels der Halteeinrichtung schwenk- und/oder verlagerbar gehalten ist. Dabei ist der Hohlkörper insbesondere lateral verlagerbar, das heißt in einer Ebene senkrecht zu einer Hochachse der Halteeinrichtung. Entkoppelt von einer lateralen Verlagerbarkeit kann auch ein Verlagern des Hohlkörpers in vertikaler Richtung, das heißt parallel zu der Hochachse der Halteeinrichtung, vorgesehen sein. Beispielsweise kann über das vertikale Verlagern des Hohlkörpers mittels der Halteeinrichtung der Hohlkörper relativ zu der Austrittsöffnung des Zerstäubungsrohrs bewegt beziehungsweise verlagert werden. Die Halteeinrichtung erlaubt auch eine Schwenkbewegung des Hohlkörpers, insbesondere um die Hochachse der Halteeinrichtung. Es kann vorgesehen sein, lediglich eine Schwenk- beziehungsweise Drehbewegung der Halteeinrichtung um die Hochachse zuzulassen, während eine Schwenk- beziehungsweise Drehbewegung um weitere Achsen durch die Halteeinrichtung verhindert ist.

Eine Weiterbildung der Erfindung sieht vor, dass das Zerstäubungsrohr mittels einer Zentriervorrichtung bezüglich einer Längsachse des Hohlkörpers in diesem zentrierbar ist. Um eine möglichst gleichmäßige Verteilung des zerstäubten Fluids auf der Innenseite des Hohlkörpers zu gewährleisten, muss ein gleichbleibender Abstand des Zerstäubungsrohrs beziehungsweise dessen Austrittsöffnung von der Innenseite des Hohlkörpers gewährleistet sein. Das bedeutet insbesondere, dass ein Abstand in radialer Richtung von der Austrittsöffnung zu der Innenseite des Hohlkörpers über einen Umfang der Innenseite im Wesentlichen gleich sein muss. Die Zentriervorrichtung sorgt dafür, dass das Zerstäubungsrohr bezüglich der Längsachse des Hohlkörpers mittig angeordnet ist. Eine Zentrierung kann entweder über eine Zentrierung des Zerstäubungsrohrs in Bezug auf den Hohlkörper oder über eine Ausrichtung des Hohlkörpers relativ zu dem Zerstäubungsrohr erreicht werden. Es erfolgt also entweder eine Verlagerung des Zerstäubungsrohrs oder des Hohlkörpers, um eine mittige Anordnung des Zerstäubungsrohrs bezüglich der Längsachse des Hohlkörpers zu erreichen. Die Zentrierung erfolgt vorzugsweise mit mechanischen Mitteln, kann aber auch mittels einer mit Sensoren verbundenen Steuerungs- beziehungsweise Regelungseinrichtung vorgenommen werden, die das Zerstäubungsrohr und/oder den Hohlkörper über Aktuatoren verlagern kann.

Eine Weiterbildung der Erfindung sieht vor, dass durch die Halteeinrichtung die Längsachse des Hohlkörpers, insbesondere achsparallel, gegenüber einer Zentrierachse der Zentriervorrichtung ausgerichtet ist. Die Halteeinrichtung kann also dazu verwendet werden, den Hohlkörper relativ zu der Zentriervorrichtung zu verlagern oder zu verschwenken. Vorzugsweise wird die Halteeinrichtung dazu verwendet, den Hohlkörper der Zentriervorrichtung zuzuführen. Dabei ist insbesondere vorgesehen, dass die Längsachse des Hohlkörpers achsparallel zu der Zentrierachse der Zentriervorrichtung ausgerichtet ist. Dadurch wird erreicht, dass während einer Verlagerung der Austrittsöffnung in dem Hohlkörper ein gleichbleibender Abstand der Austrittsöffnung beziehungsweise des Zerstäubungsrohrs zu der Innenseite des Hohlkörpers gewährleistet ist. Alternativ kann ebenso die Zentriervorrichtung bezüglich der Halteeinrichtung verlagert werden, sodass die beschriebene Ausrichtung erreicht wird.

Eine Weiterbildung der Erfindung sieht vor, dass die Verlagerungsvorrichtung an der Zentriervorrichtung vorgesehen ist. Das bedeutet, dass die Verlagerungsvorrichtung und die Zentriervorrichtung eine Baueinheit bilden. Diese Weiterbildung weist den Vorteil auf, dass eine unbeabsichtigte Verlagerung der Zentriervorrichtung gegenüber der Verlagerungsvorrichtung nicht auftreten kann, da vorzugsweise eine mechanische Verbindung zwischen Verlagerungsvorrichtung und Zentriervorrichtung ausgebildet ist.

Eine Weiterbildung der Erfindung sieht vor, dass die Zentriervorrichtung mindestens ein Zentrierelement umfasst, das einen im Wesentlichen kegelstumpfförmigen oder kegelförmigen Außenumfang aufweist. Das mindestens eine Zentrierelement definiert zumindest bereichsweise den Außenumfang der Zentriervorrichtung. Der Außenumfang dient dem Zentrieren des Hohlkörpers. Vorzugsweise bilden die Zentrierelemente einen im Wesentlichen kegelstumpfförmigen oder kegelförmigen Außenumfang aus. Zu diesem Zweck können die Zentrierelemente einen in radialer Richtung schrägen Verlauf aufweisen. Die Zentrierelemente müssen den zur Zentrierung des Hohlkörpers verwendeten Bereich des Außenumfangs der Zentriervorrichtung nicht umfänglich ununterbrochen bilden. Es kann beispielsweise vorgesehen sein, dass die Zentrierelemente aus einer Oberfläche der Zentriervorrichtung heraustreten und somit den Außenumfang definieren. Bei der Ausbildung des im Wesentlichen kegelstumpfförmigen oder kegelförmigen Außenumfangs ist es nicht notwendig, dass die Zentrierelemente auch eine Grundfläche oder eine Deckfläche des Kegelstumpfes beziehungsweise des Kegels bilden.

Es kann vielmehr vorgesehen sein, dass die Zentrierelemente lediglich in einem Bereich der Mantelfläche des Kegelstumpfes oder des Kegels vorgesehen sind.

Eine Weiterbildung der Erfindung sieht vor, dass mindestens zwei, vorzugsweise drei, Zentrierelemente vorgesehen sind, die als über den Umfang der Zentriervorrichtung verteilt angeordnete Zentrierarme ausgebildet sind. Die Zentrierarme übergreifen beispielsweise in radialer Richtung einen radialen Querschnitt der Zentriervorrichtung zumindest bereichsweise. Vorzugsweise weisen die Zentrierarme beziehungsweise die Zentrierelemente Schrägflächen auf, auf welchen der Hohlkörper sich abstützt und somit zentrierbar ist. Die Zentrierarme sind vorzugsweise dabei gleichmäßig über den Umfang der Zentriervorrichtung verteilt, um eine exakte Zentrierung des Hohlkörpers gegenüber der Zentriervorrichtung zu erreichen. Aus demselben Grund sind vorzugsweise drei Zentrierelemente vorgesehen.

Eine Weiterbildung der Erfindung sieht vor, dass die Zentriervorrichtung im Wesentlichen außerhalb eines aus der Austrittsöffnung austretenden Stroms aus zerstäubtem Fluid liegt. Das bedeutet, dass die Zentriervorrichtung in Bezug auf das Zerstäubungsrohr so angeordnet ist, dass der aus der Austrittsöffnung austretende Fluidstrom, das heißt ein Strom aus Aerosol, nicht auf die Zentriervorrichtung auftreffen kann. Die Verteilung des Aerosols auf der Innenseite des Hohlkörpers wird somit nicht beeinträcht. Möglicherweise kann (zerstäubtes) Fluid mit der Zentriervorrichtung in Verbindung kommen, allerdings nicht unmittelbar nach einem Austreten aus der Austrittsöffnung beziehungsweise vor einem Auftreffen des zerstäubten Fluids auf der Innenseite des Hohlkörpers.

Eine Weiterbildung der Erfindung sieht vor, dass das Zerstäubungsrohr eine Länge aufweist, die im Wesentlichen der axialen Erstreckung des Hohlkörpers entspricht. Um eine gleichmäßige Beschichtung eines möglichst großen Bereichs der Innenseite des Hohlkörpers erzielen zu können, ist die Austrittsöffnung vorzugsweise über einen möglichst großen Bereich des Hohlkörpers verlagerbar. Zu diesem Zweck weist das Zerstäubungsrohr in etwa dieselbe Länge auf wie der Hohlkörper, insbesondere eine axiale Erstreckung eines zu beschichtenden Bereichs der Innenseite des Hohlkörpers.

Eine Weiterbildung der Erfindung sieht vor, dass das Treibgas Luft oder Stickstoff ist. Beide Gase sind einfach handhabbar. Durch die Verfügbarkeit in großen Mengen sind sie außerdem preisgünstig.

Eine Weiterbildung der Erfindung sieht vor, dass das Fluid ein Polymer, insbesondere Silikonöl, ist oder ein Polymer aufweist. Neben einem Polymer kann alternativ eine Emulsion verwendet werden, in der ein Polymer enthalten ist, beispielsweise eine Silikonöl-Emulsion.

Eine Weiterbildung der Erfindung sieht vor, dass der Hohlkörper eine Spritze oder eine Karpule ist. Besonders bevorzugt ist die Beschichtungsvorrichtung also für Spritzen und/oder Karpulen einsetzbar. Beide weisen einen im Vergleich zu ihrer Länge geringen Durchmesser auf. Es gestaltet sich daher schwierig, zum Einen durch Einblasen eines Aerosols durch eine Öffnung des Hohlkörpers eine gleichmäßige Beschichtung der Innenseite zu erreichen, und zum Anderen, bedingt durch den geringen Durchmesser des Hohlkörpers, die Beschichtungsvorrichtung in den Hohlkörper zu verlagern. Diese Probleme sind durch die oben beschriebene Beschichtungsvorrichtung gelöst. Durch die Verwendung einer Venturi-Anordnung kann die Beschichtungsvorrichtung mit einem minimalen Platzbedarf realisiert werden, wodurch das Verlagern der Beschichtungsvorrichtung beziehungsweise des Zerstäubungsrohrs in den Hohlkörper und damit eine gleichmäßige Beschichtung der Innenseite des Hohlkörpers möglich ist.

Eine Weiterbildung der Erfindung sieht vor, dass der Hohlkörper aus Glas besteht.

Die Erfindung wird im Folgenden anhand der Zeichnung näher erläutert. Es zeigen
- Figur 1: einen Querschnitt durch die Beschichtungsvorrichtung,
- Figur 2: eine Detailansicht der Beschichtungsvorrichtung im Bereich einer Austrittsöffnung,
- Figur 3a: die Beschichtungsvorrichtung und einen zu beschichtenden Hohlkörper vor einem Zentrier- und einem Beschichtungsvorgang,
- Figur 3b: die Beschichtungsvorrichtung und den Hohlkörper während des Zentriervorgangs, und
- Figur 3c: die Beschichtungsvorrichtung mit einem in den Hohlkörper verlagerten Zerstäubungsrohr nach dem Zentrier- und vor dem Beschichtungsvorgang.

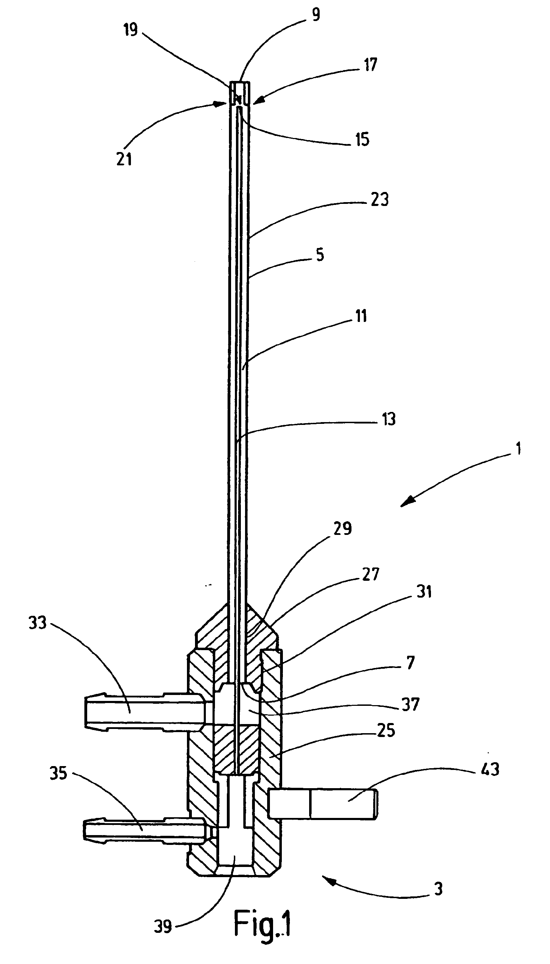

Figur 1 zeigt eine Beschichtungsvorrichtung 1, die ein Basiselement 3 und ein Zerstäubungsrohr 5 aufweist. Das Zerstäubungsrohr 5 verfügt über eine Eintrittsöffnung 7 sowie eine Austrittsöffnung 9 für ein zerstäubtes Fluid beziehungsweise Aerosol. In dem dargestellten Beispiel sind sowohl die Eintrittsöffnung 7 als auch die Austrittsöffnung 9 an den Enden des Zerstäubungsrohrs 5 angeordnet. Die Eintrittsöffnung 7 und die Austrittsöffnung 9 des Zerstäubungsrohrs 5 sind mittels eines Zerstäubungskanals 11, welcher innerhalb des Zerstäubungsrohrs 5 verläuft, fluidtechnisch gekoppelt. Das bedeutet, dass durch die Eintrittsöffnung 7 in das Zerstäubungsrohr 5 eintretendes Fluid durch den Zerstäubungskanal 11 strömt, um auf der der Eintrittsöffnung 7 abgelegenen Seite des Zerstäubungsrohrs 5 aus der Austrittsöffnung 9 auszutreten. Im Inneren des Zerstäubungskanals 11 verläuft, zumindest bereichsweise, eine Hohlnadel 13, die koaxial zu dem Zerstäubungskanal 11 angeordnet ist. Die Hohlnadel 13 verfügt über eine frontseitige Ausbringöffnung 15, die im Bereich der Austrittsöffnung 9 vorgesehen ist. Mittels der Hohlnadel 13 wird die Versorgung der Beschichtungsvorrichtung 1 mit einem unzerstäubten Fluid gewährleistet. Im Bereich der Ausbringöffnung 15 weist das Zerstäubungsrohr 5 beziehungsweise der darin angeordnete Zerstäubungskanal 11 eine Verjüngung 17 auf, in deren Bereich ein innerer Querschnitt des Zerstäubungskanals 11 bis hin zur Austrittsöffnung 9 verringert ist. Die Verjüngung 17 und die Ausbringöffnung 15 der Hohlnadel 13 sind so zueinander positioniert, dass das Zerstäubungsrohr 5 und die Hohlnadel 13 eine Venturi-Anordnung 19 bilden. Mit der Venturi-Anordnung 19 ist eine Anordnung bezeichnet, bei der die Ausbringöffnung 15 für das unzerstäubte Fluid in einem Bereich 21 angeordnet ist, der einen lokal geringsten Querschnitt des Zerstäubungskanals 11 aufweist. In dem in Figur 1 dargestellten Beispiel bildet die Hohlnadel 13 gemeinsam mit der Verjüngung 17 den Bereich 21, das heißt, dass ein Außendurchmesser der Hohlnadel 13 den Querschnitt des Zerstäubungskanals 11 in Verbindung mit der Verjüngung 17 auf den lokal geringsten Querschnitt verringert. Auch die Ausbringöffnung 15 liegt in dem Bereich 21.

Alternativ zu der frontseitigen Anordnung der Austrittsöffnung 9 an dem Zerstäubungsrohr 5 kann mindestens eine Austrittsöffnung 9 in einer Außenwandung 23 des Zerstäubungsrohrs 5 vorgesehen sein (nicht dargestellt). Beispielsweise können mindestens zwei Austrittsöffnungen 9 in der Außenwandung 23 vorgesehen sein, wobei sich in einer bevorzugten Anordnung jeweils zwei Austrittsöffnungen 9 einander diametral gegenüberliegen. Es sind aber auch mehr als zwei - vorzugsweise gleichmäßig - über den Umfang der Außenwandung 23 möglich.

Das Basiselement 3 der Beschichtungsvorrichtung 1 weist einen Anschlusskörper 25 und ein das Zerstäubungsrohr 5 haltendes Einschraubelement 27 auf. Das Zerstäubungsrohr 5 ist dabei in einer in Längsrichtung des Einschraubelements 27 vorgesehenen Bohrung gehalten. Dies kann beispielsweise dadurch realisiert werden, dass das Einschraubelement 27 als Klemmkörper ausgebildet ist, der durch Einschrauben in ein Gewinde 31 des Anschlusskörpers 25 eine Klemm- beziehungsweise Quetschwirkung auf das Zerstäubungsrohr 5 ausübt und dieses somit fixiert. An dem Anschlusskör per 25 sind zwei Anschlussstutzen 33 und 35 vorgesehen. Über den Anschlussstutzen 33 erfolgt ein Anschluss der Beschichtungsvorrichtung 1 an eine nicht dargestellte Treibgas-Versorgung, während über den Anschlussstutzen 35 das unzerstäubte Fluid zuführbar ist. Der Anschlussstutzen 33 steht in Fluidkommunikation mit einer Sammelkammer 37, die als Beruhigungskammer zur Beruhigung des Treibgases dient, bevor dieses über die mit der Sammelkammer 37 verbundene Eintrittsöffnung 7 in das Zerstäubungsrohr 5 eintritt. Der Anschlussstutzen 35 steht in Verbindung mit einer Sammelkammer 39, über die eine Verbindung zu der Hohlnadel 13 hergestellt ist. Die Sammelkammern 37 und 39 sind durch ein als Stopfen 41 ausgebildetes Trennelement voneinander separiert. Das bedeutet, dass keine Fluidverbindung zwischen der Sammelkammer 37 und der Sammelkammer 39 vorliegt, das Treibgas zunächst also nicht in Kontakt mit dem unzerstäubten Fluid treten kann. Die Beschichtungsvorrichtung 1 weist weiterhin eine Haltevorrichtung 43 auf, die an dem Anschlusskörper vorgesehen ist und zur Befestigung an einem weiteren, nicht dargestellten Bauteil dient.

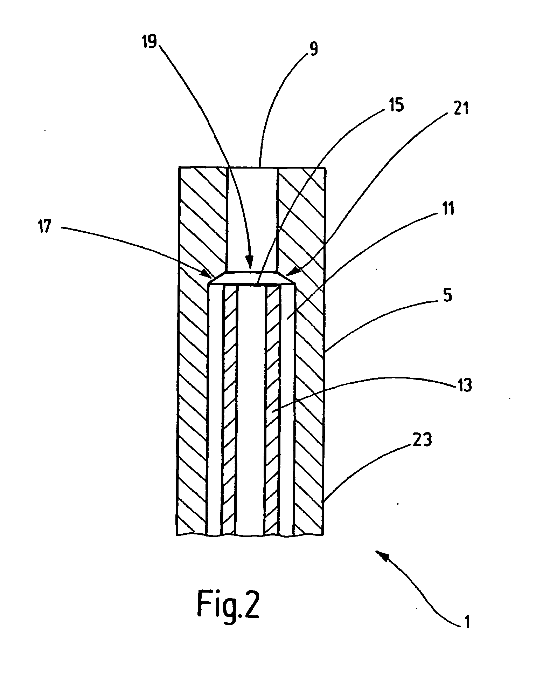

Die Figur 2 zeigt eine Detailansicht der Beschichtungsvorrichtung 1 im Bereich der Austrittsöffnung 9 beziehungsweise der Venturi-Anordnung 19. Es wird deutlich, dass die Venturi-Anordnung 19 im Bereich der Austrittsöffnung 9 angeordnet ist. Das bedeutet, dass die Venturi-Anordnung 19 in Bezug auf eine Länge des Zerstäubungsrohrs 5 nahe der Austrittsöffnung 9 angeordnet ist. In dem Bereich des Zerstäubungsrohrs 5, in welchem die Hohlnadel 13 angeordnet ist, liegt ein Querschnitt vor, welcher von dem Treibgas durchströmbar ist. Im Bereich der Venturi-Anordnung 19, also in dem Bereich, in welchem die Verjüngung 17 und die Ausbringöffnung 15 der Hohlnadel 13 angeordnet sind, liegt ein im Vergleich zu dem genannten, stromaufseitig gelegenen Querschnitt, ein verringerter Querschnitt vor. Das Treibgas wird somit beschleunigt, worauf der statische Druck des Treibgases abfällt. Weiter stromabwärts entfällt die durch die Hohlnadel 13 bewirkte Querschnittsverringerung des Zerstäubungskanals 11, womit sich der Querschnitt wieder erweitert. Das bedeutet, dass die Ausbringöffnung 15 im Bereich des geringsten Querschnitts angeordnet ist. Im Sinne des engsten Querschnitts ist lediglich der lokal geringste Querschnitt zu verstehen. Es ist durchaus möglich, dass der Zerstäubungskanal 11 abseits der Venturi-Anordnung 19 einen noch geringeren Querschnitt aufweist. Die Anordnung der Ausbringöffnung 15 im Bereich des geringsten Querschnitts bedeutet, dass an dieser Stelle ein verminderter Druck vorliegt. Dies folgt beispielsweise aus der Benoulli-Gleichung. Durch den geringen Druck kann das unzerstäubte Fluid durch die Ausbringöffnung 15 aus der Hohlnadel 13 austreten, ohne dass dem unzerstäubten Fluid ein hoher Druck aufgeprägt werden muss. Im Bereich der Venturi-Anordnung 19 liegen durch die unterschiedlichen Querschnitte somit eine Kontraktion des Zerstäubungskanals 11 und ein anschließender Diffusor vor, in dessen Verlauf der Querschnitt wieder vergrößert wird. Die Venturi-Anordnung 19 bewirkt eine feine Zerstäubung des unzerstäubten Fluids mittels des Treibgases.

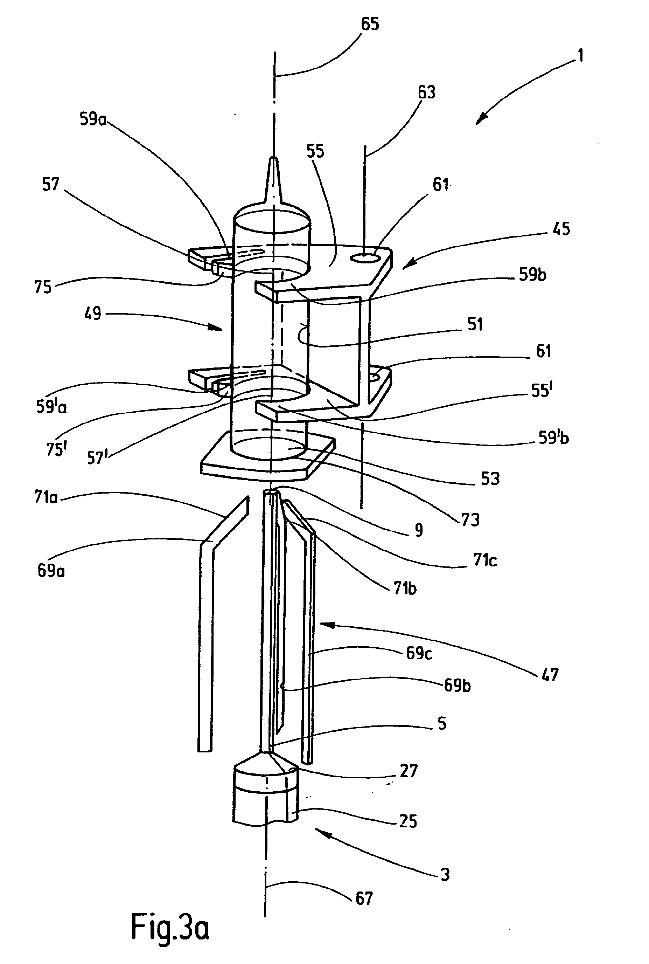

Die Figur 3a zeigt die Beschichtungsvorrichtung 1, die neben dem Zerstäubungsrohr 5 und dem Basiselement 3 eine Halteeinrichtung 45 und eine Zentriervorrichtung 47 aufweist. In der Halteeinrichtung 45 ist ein Hohlkörper 49, in diesem Beispiel ein Spritzenzylinder, gehalten. Der Hohlkörper 49 weist eine Innenseite 51 sowie eine Öffnung 53 auf. Die Halteeinrichtung 45 weist in dem in Figur 3a dargestellten Beispiel zwei Klemmböden 55, 55' auf, die jeweils eine Aussparung 57, 57' aufweisen, die von jeweils zwei Haltenasen 59a, 59b, 59'a, 59'b zumindest bereichsweise umfangen ist. In den Aussparungen 57 ist der Hohlkörper 49 gehalten. Jeweils eine der Haltenasen 59a, 59b, 59'a, 59'b ist als elastische Haltenase ausgebildet und kann bei Kraftbeaufschlagung in radialer Richtung in Bezug auf die Aussparung 57, 57' nach außen ausweichen. Die elastischen Haltenasen 59a, 59b, 59'a, 59'b erlauben ein Einbringen des Hohlkörpers 49 in die Aussparung 57, 57' und ein anschließendes Halten des Hohlkörpers 49. Die beiden Klemmböden 55, 55' sind in vertikaler Richtung voneinander in einem Abstand zueinander angeordnet. Die Halteeinrichtung 45 kann mittels Bohrungen 61 beispielsweise auf nicht dargestellten Stiften einer nicht dargestellten, benachbarten Vorrichtung angeordnet sein. Zentrisch durch die Bohrungen 61 verläuft eine Längsachse 63 der Halteeinrichtung 45. Sie erlaubt ein freies Verschwenken des Hohlkörpers 49 um die Längsachse 63. Weiterhin ist vorgesehen, dass der Hohlkörper 49 mittels der Halteeinrichtung 45 in lateraler Richtung, das heißt in einer Ebene senkrecht zu der Längsachse 63, verschiebbar ist. Die Halteeinrichtung 45 hält den Hohlkörper 49 derart, dass eine Längsachse 65 des Hohlkörpers parallel zu der Längsachse 63 der Halteeinrichtung verläuft. Es ist auch vorgesehen, dass die Halteeinrichtung 45 so angeordnet ist, dass die Längsachse 63 der Halteeinrichtung 45 sowie die Längsachse 65 des Hohlkörpers parallel zu einer Zentrierachse 67 der Zentriervorrichtung 47 verlaufen.

Die Zentriervorrichtung 47 weist drei Zentrierelemente 69 auf, die hier als Zentrierarme ausgebildet sind. Die Zentrierelemente 69a, 69b, 69c sind so angeordnet, dass sie in keiner Position innerhalb eines aus der Austrittsöffnung 9 des Zerstäubungsrohrs 5 austretenden Stroms aus zerstäubten Fluid liegen. Damit wird verhindert, dass die Zentriervorrichtung 47 den Strom aus zerstäubten Fluid und damit eine gleichmäßige Beschichtung der Innenseite 51 des Hohlkörpers 49 beeinflusst. Mittels der Zentriervorrichtung 47 beziehungsweise der Zentrierelemente 69a, 69b, 69c kann der Hohlkörper 49 so angeordnet werden, dass die Zentrierachse 47 und die Längsachse 65 des Hohlkörpers 49 übereinstimmen. In diesem Fall ist auch die Austrittsöffnung 9 des Zerstäubungsrohrs 5 zentrisch in Bezug zu dem Hohlkörper 49 positioniert. Die Positionierung des Hohlkörpers 49 wird über Schrägflächen 71a, 71b, 71c der Zentrierelemente 69 vorgenommen. Diese verlaufen in Bezug auf die Zentrierachse 67 radial nach innen und nach oben und bilden somit einen kegelstumpfförmigen oder kegelförmigen Außenumfang der Zentriervorrichtung 47 aus. In dem dargestellten Beispiel erstrecken sich die Schrägflächen 71a, 71b, 71c lediglich abschnittsweise auf die Zentrierachse 67 beziehungsweise das Zerstäubungsrohr 5 zu. Es ist also ein kegelstumpfförmiger Außenumfang der Zentriervorrichtung 47, jedoch ohne eine Grund- beziehungsweise Deckfläche des Kegelstumpfs, ausgebildet. Die Erstreckung der Schrägflächen 71 a, 71b, 71c nach innen erfolgt so weit, dass der Abstand von Enden der Zentrierelemente 69a, 69b, 69c zueinander geringer ist, als ein Durchmesser der Öffnung 53 des Hohlkörpers 49. Damit kann während eines Zentriervorgangs eine Kante 73 der Öffnung 53 des Hohlkörpers 49 in Auflagekontakt mit den Schrägflächen 71a, 71b, 71c beziehungsweise den Zentrierelementen 69a, 69b, 69c der Zentriervorrichtung 47 treten.

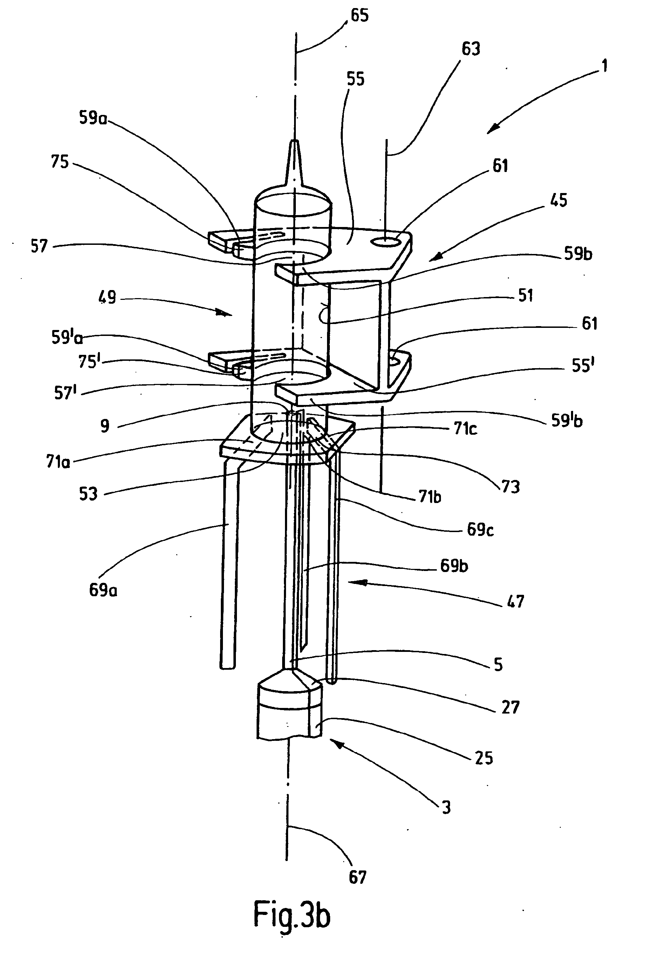

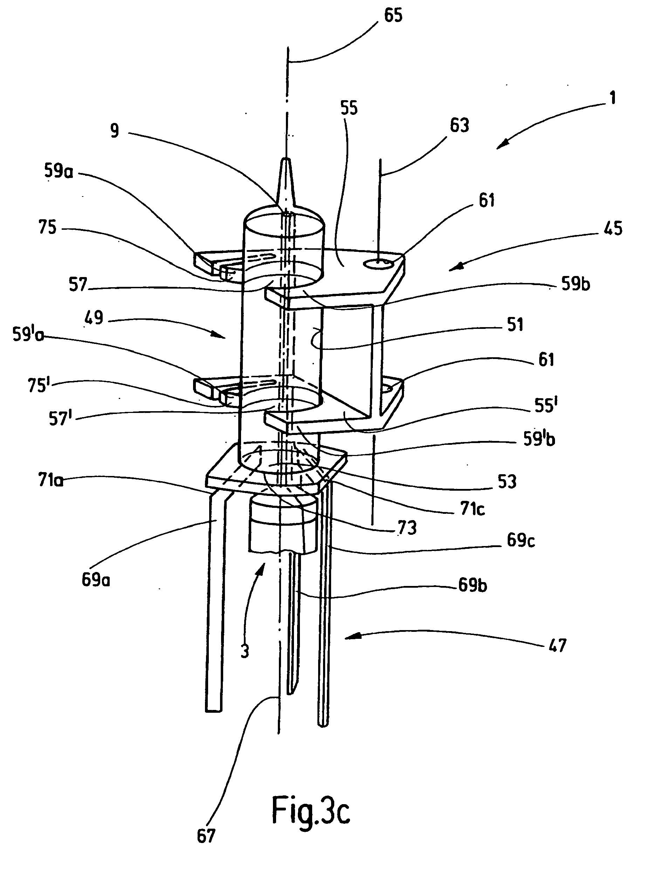

Die Funktion der Beschichtungsvorrichtung 1 wird im Folgenden anhand der Figuren 3a, 3b und 3c beschrieben: Figur 3a zeigt die Beschichtungsvorrichtung 1 und den zu beschichtenden Hohlkörper 49 vor dem Zentriervorgang und dem Beschichtungsvorgang. Zunächst werden die Beschichtungsvorrichtung 1 und der Hohlkörper 49 bereitgestellt, wobei der Hohlkörper 49 in die Aussparung 57 eingeclipst wird. Dies erfolgt dadurch, dass der Hohlkörper 49 in den nicht von den Haltenasen 59a, 59b, 59'a, 59'b umschlossenen Bereich eingedrückt wird. Dadurch wirkt der Hohlkörper 49 über schräg angestellte Flächen 75, 75' eine Kraft in radialer Richtung auf die Haltenasen 59a, 59b, 59'a, 59'b aus, worauf diese zurückweichen und der Hohlkörper 49 in die Aussparung 57, 57' eingebracht werden kann. Durch eine elastische Ausbildung der Haltenasen 59a, 59b, 59'a, 59'b oder durch eine Federwirkung einer nicht dargestellten Feder werden die Haltenasen nach dem Einbringen des Hohlkröpers 49 wieder in ihre Ausgangsposition gebracht. Damit ist der Hohlkörper 49 zumindest bereichsweise von den Haltenasen 59a, 59b, 59'a, 59'b in der Aussparung 57, 57' eingeschlossen und wird durch Clipswirkung darin gehalten. Damit ist der Hohlkörper 49 so angeordnet, dass seine Längsache 65 parallel zu der Längsachse 63 der Halteeinrichtung 45 sowie der Zentrierachse 67 der Zentriervorrichtung 47 verläuft. Folglich ist die Längsachse 65 des Hohlkörpers 49 bereits im Wesentlichen parallel zu dem Zerstäubungsrohr 5 angeordnet. In dem in Figur 3a dargestellten Zustand befindet sich die Beschichtungsvorrichtung 1 in einem Ausgangszustand. Das bedeutet, dass das Zerstäubungsrohr 5 noch nicht mittels einer nicht dargestellten Verlagerungsvorrichtung verlagert wurde, sondern so angeordnet ist, dass sich die Austrittsöffnung 9 in etwa auf Höhe der maximalen Erstreckung der Zentriervorrichtung 47 in vertikaler Richtung befindet. Das bedeutet, dass die Austrittsöffnung 9 nicht oberhalb der Zentriervorrichtung 47 angeordnet ist. Der Hohlkörper 49 ist durch die Halteeinrichtung 45 um die Längsachse 63 der Halteeinrichtung 45 drehbar gelagert.

Im Folgenden wird der Hohlkörper 49 mittels der Halteeinrichtung 45 in Richtung der Zentriervorrichtung 47 verlagert, sodass die Längsachse 65 des Hohlkörpers 49 näherungsweise mit der Zentrierachse 67 übereinstimmt. Alternativ kann auch die Zentriervorrichtung 47 verlagert werden, ohne dass die Halteeinrichtung 45 bewegt wird, sodass die vorstehend beschriebene Ausrichtung erreicht wird. Anschließend wird entweder die Halteeinrichtung 45 abgesenkt oder die Zentriervorrichtung 47 angehoben. Damit liegt der in Figur 3b abgebildete Zustand vor.

Die Figur 3b zeigt die Beschichtungsvorrichtung 1 und den Hohlkörper 49 während des Zentriervorgangs. Durch das Anheben der Zentriervorrichtung 47 beziehungsweise der Absenkung der Halteeinrichtung 45 tritt die Kante 73 des Hohlkörpers in Auflagekontakt mit den Schrägflächen 71a, 71b, 71c der Zentriervorrichtung 47. Entweder die Zentriervorrichtung 47 oder die Halteeinrichtung 45 sind lateral beweglich, das heißt in einer Ebene senkrecht zu der Zentrierachse 67 beziehungsweise der Längsachse 63 verschiebbar. Damit kann sich der Hohlkörper 49, sobald er mit der Kante 73 auf die Zentrierelemente 69a, 69b, 69c aufgesetzt wird, bedingt durch die Schwerkraft beziehungsweise eine durch die Halteeinrichtung 45 in vertikaler Richtung nach unten oder durch die Zentriervorrichtung 47 in vertikaler Richtung nach oben aufgeprägte Kraft, so ausrichten, dass seine Längsachse 65 mit der Zentrierachse 67 übereinstimmt. Dabei gleitet die Kante 73 über die Schrägflächen 71a, 71b, 71c der Zentrielemente 69a, 69b, 69c.

Sobald alle Zentrierelemente 69a, 69b, 69c in flächigem Kontakt mit der Kante 73 stehen, kann angenommen werden, dass die gewünschte Anordnung des Hohlkörpers 49 vorliegt. Das bedeutet, dass der Hohlkörper 49 so ausgerichtet ist, dass seine Längsachse 65 mit der Zentrierachse 67 beziehungsweise einer Längsachse des Zerstäubungsrohrs 5 übereinstimmt. Ein Abstand von der Austrittsöffnung 9 zu in einer Vertikalebene angeordneten Punkten der Innenseite 51 des Hohlkörpers 49 ist somit für alle Punkte gleich. Auf diese Weise kann während eines nachfolgenden Beschichtungsvorgangs eine besonders homogene Beschichtung der Innenseite 51 erreicht werden. Sobald der anhand von Figur 3b beschriebene Zentriervorgang abgeschlossen ist, wird die Beschichtung der Innenseite 51 des Hohlkörpers 49 vorgenommen.

Dazu wird, wie in Figur 3c gezeigt, zunächst das Zerstäubungsrohr 5 beziehungsweise die Austrittsöffnung 9 in den Hohlkörper 59 hineinverlagert. Anschließend wird der Beschichtungsvorgang gestartet, das heißt sowohl Treibgas als auch unzerstäubtes Fluid in die Beschichtungsvorrichtung 1 eingebracht. Währenddessen wird die Austrittsöffnung 9 vertikal nach unten bewegt, und somit das Zerstäubungsrohr 5 aus dem Hohlkörper 49 herausbewegt. Während des Beschichtungsvorgangs beziehungsweise des Herausziehens des Zerstäubungsrohrs 5 aus dem Hohlkörper 49 liegt somit für einen Großteil der Punkte auf der Innenseite 51 derselbe Abstand zu der Austrittsöffnung 9 vor.

Alternativ kann auch vorgesehen sein, dass der Beschichtungsvorgang während des Einbringens des Zerstäubungsrohrs 5 in den Hohlkörper 49 durchgeführt wird. Das heißt, dass nicht zunächst das Zerstäubungsrohr 5 in den Hohlkörper 49 verlagert wird und erst während eines Herausziehens die Beschichtung aufgebracht wird. Möglich sind auch mehrere Beschichtungsdurchläufe. Eine Beschichtung kann beispielsweise sowohl während des Einbringens des Zerstäubungsrohrs 5 in den Hohlkörper 49, als auch während des Herausbewegens erfolgen. Während des Beschichtungsvorgangs sind vorzugsweise die Halteeinrichtung 45 und die Zentriervorrichtung 47 aneinander fixiert. Damit ist auch eine sichere Fixierung des Hohlkörpers 49 in Bezug auf das Zerstäubungsrohr 5 beziehungsweise die Austrittsöffnung 9 gegeben. Anschließend an den Beschichtungsvorgang wird das Zerstäubungsrohr 5 aus dem Hohlkörper 49 entfernt und entweder die Halteeinrichtung 45 nach oben oder die Zentriervorrichtung 47 nach unten bewegt, sodass der Hohlkörper 49 nicht mehr in Verbindung mit der Zentriervorrichtung 47 steht. Anschließend wird der Hohlkörper 49 von der Halteeinrichtung 45 entfernt, wiederum durch Aufprägen einer Kraft auf den Hohlkörper 49 in Richtung des Bereichs, der nicht von den Haltenasen 59a, 59b, 59'a, 59'b eingefasst ist. Nachfolgend können weitere Bearbeitungsschritte an dem Hohlkörper 49 vorgenommen werden.

## Patentansprüche

1. Beschichtungsvorrichtung zur Beschichtung einer Innenseite eines Hohlkörpers mit einem zerstäubten Fluid, **gekennzeichnet durch**,
- mindestens ein einen Zerstäubungskanal (11) einschließendes Zerstäubungsrohr (5), in das unter einem Überdruck stehendes Treibgas zum Zerstäuben eines unzerstäubten Fluids einleitbar ist, und das mindestens eine Austrittsöffnung (9) aufweist, und
- mindestens eine eine Ausbringöffnung (15) aufweisende Hohlnadel (13) für das unzerstäubte Fluid, die mit dem Zerstäubungskanal (11) zusammenwirkt und im Wesentlichen koaxial zu diesem angeordnet ist, wobei
- das Zerstäubungsrohr (5) und die Hohlnadel (13) eine Venturi-Anordnung (19) bilden,
wobei die mindestens eine Austrittsöffnung (9) über zumindest einen Teil einer axialen Erstreckung der Innenseite (51) des Hohlkörpers (49) mittels einer Verlagerungsvorrichtung verlagerbar ist, das Zerstäubungsrohr (5) mittels einer Zentriervorrichtung (47) bezüglich einer Längsachse (65) des Hohlkörpers (49) in diesem zentrierbar ist, und dass der Hohlkörper (49) eine Spritze oder eine Karpule ist.

2. Beschichtungsvorrichtung nach Anspruch 1, **dadurch gekennzeichnet, dass** die Hohlnadel (13) zumindest bereichsweise im Zerstäuburigskanal (11) angeordnet ist.

3. Beschichtungsvorrichtung nach einem oder mehreren der vorhergehenden Ansprüche, **dadurch gekennzeichnet, dass** eine Verjüngung (17) des Zerstäubungskanals (11) vorgesehen ist.

4. Beschichtungsvorrichtung nach einem oder mehreren der vorhergehenden Ansprüche, **dadurch gekennzeichnet, dass** die Ausbringöffnung (15) der Hohlnadel (13) in einem Bereich der Verjüngung (17) angeordnet ist.

5. Beschichtungsvorrichtung nach einem oder mehreren der vorhergehenden Ansprüche, **dadurch gekennzeichnet, dass** die Ausbringöffnung (15) in einem Bereich der Austrittsöffnung (9) vorgesehen ist.

6. Beschichtungsvorrichtung nach einem oder mehreren der vorhergehenden Ansprüche, **dadurch gekennzeichnet, dass** die mindestens eine Austrittsöffnung (9) frontseitig an dem Zerstäubungsrohr (5) vorgesehen ist.

7. Beschichtungsvorrichtung nach einem oder mehreren der vorhergehenden Ansprüche, **dadurch gekennzeichnet, dass** die mindestens eine Austrittsöffnung (9) in einer Außenwandung (23) des Zerstäubungsrohrs (5) vorgesehen ist.

8. Beschichtungsvorrichtung nach einem oder mehreren der vorhergehenden Ansprüche, **dadurch gekennzeichnet, dass** mindestens zwei Austrittsöffnungen (9) vorgesehen sind, die in der Außenwandung (23) des Zerstäubungsrohrs (5) vorgesehen sind, und die über einen Umfang des Zerstäubungsrohrs (5) verteilt, insbesondere einander diametral gegenüberliegend, angeordnet sind.

9. Beschichtungsvorrichtung nach einem oder mehreren der vorhergehenden Ansprüche, **dadurch gekennzeichnet, dass** der Hohlkörper (49) während der Beschichtung von einer, insbesondere klammerförmigen, Halteeinrichtung (45) gehalten ist.

10. Beschichtungsvorrichtung nach einem oder mehreren der vorhergehenden Ansprüche, **dadurch gekennzeichnet, dass** der Hohlkörper (49) in die Halteeinrichtung (45) einclipsbar beziehungsweise durch Clipswirkung darin gehalten ist.

11. Beschichtungsvorrichtung nach einem oder mehreren der vorhergehenden Ansprüche, **dadurch gekennzeichnet, dass** der Hohlkörper (49) mittels der Halteeinrichtung (45) schwenk- und/oder verlagerbar gehalten ist.

12. Beschichtungsvorrichtung nach einem oder mehreren der vorhergehenden Ansprüche, **dadurch gekennzeichnet, dass** durch die Halteeinrichtung (45) die Längsachse (65) des Hohlkörpers (49), insbesondere achsparallel, an einer Zentrierachse (67) der Zentriervorrichtung (47) ausgerichtet ist.

13. Beschichtungsvorrichtung nach einem oder mehreren der vorhergehenden Ansprüche, **dadurch gekennzeichnet, dass** die Verlagerungsvorrichtung an der Zentriervorrichtung (47) vorgesehen ist.

14. Beschichtungsvorrichtung nach einem oder mehreren der vorhergehenden Ansprüche, **dadurch gekennzeichnet, dass** die Zentriervorrichtung (47) mindestens ein Zentrierelement (69) aufweist, das einen im Wesentlichen kegelstumpfförmigen oder kegelförmigen Außenumfang bildet.

15. Beschichtungsvorrichtung nach einem oder mehreren der vorhergehenden Ansprüche, **dadurch gekennzeichnet, dass** mindestens zwei, vorzugsweise drei, Zentrierelemente (69a,69b,69c) vorgesehen sind, die als über den Umfang der Zentriervorrichtung (47) verteilt angeordnete Zentrierarme ausgebildet sind.

16. Beschichtungsvorrichtung nach einem oder mehreren der vorhergehenden Ansprüche, **dadurch gekennzeichnet, dass** die Zentriervorrichtung (47) im Wesentlichen außerhalb eines aus der Austrittsöffnung (9) austretenden Stroms aus zerstäubtem Fluid liegt.

17. Beschichtungsvorrichtung nach einem oder mehreren der vorhergehenden Ansprüche, **dadurch gekennzeichnet, dass** das Zerstäubungsrohr (5) eine Länge aufweist, die im Wesentlichen der axialen Erstreckung des Hohlkörpers (49) entspricht.

18. Beschichtungsvorrichtung nach einem oder mehreren der vorhergehenden Ansprüche, **dadurch gekennzeichnet, dass** das Treibgas Luft oder Stickstoff ist.

19. Beschichtungsvorrichtung nach einem oder mehreren der vorhergehenden Ansprüche, **dadurch gekennzeichnet, dass** das Fluid ein Polymer, insbesondere Silikonöl, ist oder ein Polymer aufweist.

20. Beschichtungsvorrichtung nach einem oder mehreren der vorhergehenden Ansprüche, **dadurch gekennzeichnet, dass** der Hohlkörper (49) aus Glas besteht.

## Claims

1. Coating device for coating an inside of a hollow body with an atomized fluid, **characterized by**
- at least one atomizing tube (5) enclosing an atomizing channel (11), in which atomizing tube propellant gas being under over-pressure for atomizing an unatomized fluid can be introduced and which has at least one outlet opening (9), and
- at least one hollow needle (13) having a discharge opening (15) for the unatomized fluid, which interacts with the atomizing channel (11) and is arranged essentially coaxially thereto, wherein
- the atomizing tube (5) and the hollow needle (13) form a Venturi arrangement (19),
wherein the at least one outlet opening (9) can be displaced over at least a part of an axial extension of the inside (51) of the hollow body (49) by means of a displacement device, the atomizing tube (5) can be centred by means of a centring device (47) with regard to a longitudinal axis (65) of the hollow body (49) therein and that the hollow body (49) is a syringe or a carpule.

2. Coating device according to claim 1, **characterized in that** the hollow needle (13) is arranged at least in some regions in the atomizing channel (11).

3. Coating device according to one or more of the preceding claims, **characterized in that** a tapering (17) of the atomizing channel (11) is provided.

4. Coating device according to one or more of the preceding claims, **characterized in that** the discharge opening (15) of the hollow needle (13) is arranged in a region of the tapering (17).

5. Coating device according to one or more of the preceding claims, **characterized in that** the discharge opening (15) is provided in a region of the outlet opening (9).

6. Coating device according to one or more of the preceding claims, **characterized in that** the at least one outlet opening (9) is provided on the front of the atomizing tube (5).

7. Coating device according to one or more of the preceding claims, **characterized in that** the at least one outlet opening (9) is provided in an outer wall (23) of the atomizing tube (5).

8. Coating device according to one or more of the preceding claims, **characterized in that** at least two outlet openings (9) are provided, which are provided in the outer wall (23) of the atomizing tube (5) and which are arranged distributed over a circumference of the atomizing tube (5), in particular lying diametrically opposite one another.

9. Coating device according to one or more of the preceding claims, **characterized in that** the hollow body (49) is held by an, in particular clamp-shaped, holding device (45) during the coating.

10. Coating device according to one or more of the preceding claims, **characterized in that** the hollow body (49) can be clipped into the holding device (45) or is held therein by clipping effect.

11. Coating device according to one or more of the preceding claims, **characterized in that** the hollow body (49) is held by means of the holding device (45) in a pivoting and/or displaceable manner.

12. Coating device according to one or more of the preceding claims, **characterized in that** the longitudinal axis (65) of the hollow body (49) is aligned, in particular in an axially parallel manner, to a centring axis (67) of the centring device (47) by the holding device (45).

13. Coating device according to one or more of the preceding claims, **characterized in that** the displacement device is provided on the centring device (47).

14. Coating device according to one or more of the preceding claims, **characterized in that** the centring device (47) has at least one centring element (69), which forms an essentially frustoconical or conical outer circumference.

15. Coating device according to one or more of the preceding claims, **characterized in that** at least two, preferably three, centring elements (69a, 69b, 69c) are provided, which are embodied as centring arms arranged distributed over the circumference of the centring device (47).

16. Coating device according to one or more of the preceding claims, **characterized in that** the centring device (47) lies essentially outside a flow of atomized fluid exiting from the outlet opening (9).

17. Coating device according to one or more of the preceding claims, **characterized in that** the atomizing tube (5) has a length that corresponds essentially to the axial extension of the hollow body (49).

18. Coating device according to one or more of the preceding claims, **characterized in that** the propellant gas is air or nitrogen.

19. Coating device according to one or more of the preceding claims, **characterized in that** the fluid is a polymer, in particular silicone oil, or has a polymer.

20. Coating device according to one or more of the preceding claims, **characterized in that** the hollow body (49) is made of glass.

## Revendications

1. Dispositif de revêtement pour revêtir une face interne d'un corps creux avec un fluide pulvérisé sur, **caractérisé par,**
- au moins un tube de pulvérisation (5) intégrant un canal de pulvérisation (11) et présentant au moins une ouverture de sortie (9), un gaz propulseur soumis à une surpression pouvant être introduit dans ledit tube de pulvérisation pour pulvériser un fluide non pulvérisé, et
- au moins une aiguille creuse (13) présentant une ouverture de distribution (15) pour le fluide non pulvérisé, ladite aiguille creuse coopérant avec le canal de pulvérisation (11) et étant disposée sensiblement coaxialement à celui-ci, et dont
- le tube de pulvérisation (5) et l'aiguille creuse (13) forment un système venturi (19),
et que l'au moins une ouverture de sortie (9) soit déplaçable sur au moins une partie d'une étendue axiale de la face interne (51) du corps creux (49) à l'aide d'un dispositif de déplacement, que le tube de pulvérisation (5) soit centrable à l'aide d'un dispositif de centrage (47) dans le corps creux (49) par rapport à un axe longitudinal (65) de celui-ci, et que le corps creux (49) soit une seringue ou une carpule.

2. Dispositif de revêtement selon la revendication 1, **caractérisé en ce, que** l'aiguille creuse (13) soit agencée au moins par zones dans le canal de pulvérisation (11).

3. Dispositif de revêtement selon l'une ou plusieurs des revendications précédentes, **caractérisé en ce, qu'**un rétrécissement (17) du canal de pulvérisation (11) soit prévu.

4. Dispositif de revêtement selon l'une ou plusieurs des revendications précédentes, **caractérisé en ce, que** l'ouverture de distribution (15) de l'aiguille creuse (13) soit située dans une zone du rétrécissement (17).

5. Dispositif de revêtement selon l'une ou plusieurs des revendications précédentes, **caractérisé en ce, que** l'ouverture de distribution (15) soit prévue dans une zone de l'ouverture de sortie (9).

6. Dispositif de revêtement selon l'une ou plusieurs des revendications précédentes, **caractérisé en ce, que** l'au moins une ouverture de sortie (9) soit prévue sur la partie frontale du tube de pulvérisation (5).

7. Dispositif de revêtement selon l'une ou plusieurs des revendications précédentes, **caractérisé en ce, que** l'au moins une ouverture de sortie (9) soit prévue sur une paroi extérieure (23) du tube de pulvérisation (5).

8. Dispositif de revêtement selon l'une ou plusieurs des revendications précédentes, **caractérisé en ce, qu'**au moins deux ouvertures de sortie (9) soient prévues sur la paroi extérieure (23) du tube de pulvérisation (5) et qu'elles soient réparties sur un périmètre du tube de pulvérisation (5), en étant notamment agencées de manière diamétralement opposée.

9. Dispositif de revêtement selon l'une ou plusieurs des revendications précédentes, **caractérisé en ce, que** le corps creux (49) soit maintenu pendant l'application du revêtement par un dispositif de support (45) possédant une forme de clip.

10. Dispositif de revêtement selon l'une ou plusieurs des revendications précédentes, **caractérisé en ce, que** le corps creux (49) soit enclipsable dans le dispositif de support (45) ou bien qu'il y soit maintenu par effet de clipsage.

11. Dispositif de revêtement selon l'une ou plusieurs des revendications précédentes, **caractérisé en ce, que** le corps creux (49) soit pivotant et/ou déplaçable à l'aide du dispositif de support (45).

12. Dispositif de revêtement selon l'une ou plusieurs des revendications précédentes, **caractérisé en ce, que**, par le dispositif de support (45), l'axe longitudinal (65) du corps creux (49) soit aligné, notamment de manière axialement parallèle, à un axe de centrage (67) du dispositif de centrage (47).

13. Dispositif de revêtement selon l'une ou plusieurs des revendications précédentes, **caractérisé en ce, que** le dispositif de déplacement soit prévu au dispositif de centrage (47).

14. Dispositif de revêtement selon l'une ou plusieurs des revendications précédentes, **caractérisé en ce, que** le dispositif de centrage (47) comporte au moins un élément de centrage (69), qui forme un pourtour extérieur essentiellement tronconique ou conique.

15. Dispositif de revêtement selon l'une ou plusieurs des revendications précédentes, **caractérisé en ce, que** soient prévus au moins deux, de préférence trois, éléments de centrage (69a, 69b, 69c), qui sont conçus en tant que bras de centrage agencés de manière qu'ils soient répartis sur le pourtour du dispositif de centrage (47).

16. Dispositif de revêtement selon l'une ou plusieurs des revendications précédentes, **caractérisé en ce, que** le dispositif de centrage (47) soit essentiellement situé à l'extérieur d'un flux de fluide pulvérisé sortant de l'ouverture de sortie (9).

17. Dispositif de revêtement selon l'une ou plusieurs des revendications précédentes, **caractérisé en ce, que** le tube de pulvérisation (5) présente une longueur, qui correspond essentiellement à l'étendue axiale du corps creux (49).

18. Dispositif de revêtement selon l'une ou plusieurs des revendications précédentes, **caractérisé en ce, que** le gaz propulseur soit de l'air ou de l'azote.

19. Dispositif de revêtement selon l'une ou plusieurs des revendications précédentes, **caractérisé en ce, que** le fluide soit un polymère, notamment de l'huile de silicone, ou qu'il comporte un polymère.

20. Dispositif de revêtement selon l'une ou plusieurs des revendications précédentes, **caractérisé en ce, que** le corps creux (49) soit en verre.
